# EUROPÄISCHE PATENTSCHRIFT

(11) **EP 0 442 401 B1**
(45) Veröffentlichungstag und Bekanntmachung des Hinweises auf die Patenterteilung: **27.04.1994**
(21) Anmeldenummer: 91101829.9
(22) Anmeldetag: 09.02.1991
(51) Int. Cl.: C07D 473/04

(54) **Verfahren zur Gewinnung annähernd fluoreszenzfreier Xanthine**
Process for the synthesis of essentially non-fluorescent xanthines
Procédé pour la récupération de xanthines sans fluorescence essentielle

(30) Priorität: 15.02.1990 DE 4004618
(43) Veröffentlichungstag der Anmeldung: 21.08.1991
(73) Patentinhaber: HOECHST AKTIENGESELLSCHAFT, 65926 Frankfurt am Main (DE)
(72) Erfinder: Knieps, Rudolf, Dr., W-6233 Kelkheim-Taunus (DE); Jaenicke, Ottmar, Dr., W-6233 Kelkheim-Taunus (DE); Schönfeld, Walter, Dr., W-6200 Wiesbaden (DE)

(56) Entgegenhaltungen:
- DD-A- 21 220
- DE-C- 138 444
- GB-A- 4 959
- GB-A- 302 207

## Beschreibung

Der Xanthin-"Grundkörper" ist die Verbindung der Formel

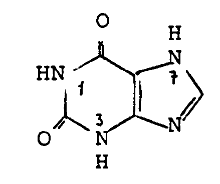

Das unsubstituierte Xanthin und substituierte Xanthine sind hauptsächlich Vor- bzw. Zwischenprodukte zur Herstellung von Arzneimitteln; verschiedene substituierte Xanthine sind auch bereits selbst wertvolle Arzneimittel-Wirkstoffe. Ein bekannter derartiger Arzneimittel-Wirkstoff ist z.B. die Verbindung Pentoxifyllin (= 1-(5-Oxohexyl)-3,7-dimethylxanthin), welche das wirksame Prinzip verschiedener Arzneimittel gegen Durchblutungsstörungen ist. Ein pentoxifyllinhaltiges Arzneimittel zur Behandlung von Durchblutungsstörungen ist z.B. das von der Firma Albert-Roussel Pharma GmbH, Wiesbaden (Bundesrepublik Deutschland) vertriebene Produkt ^{(R)}Trental.

Eine bevorzugte Synthese des Xanthin-Ringsystems ist die sog. "modifizierte Traube-Synthese" (Ullmann's Enzyklopädie der Technischen Chemie, 4. Aufl., Band 19 (1980) S. 579). Die Synthese geht aus von Harnstoff bzw. Harnstoffderivaten und Cyanessigsäure; sie stellt sich z.B. für die Herstellung des 3-Methylxanthins wie folgt dar (schematisch):

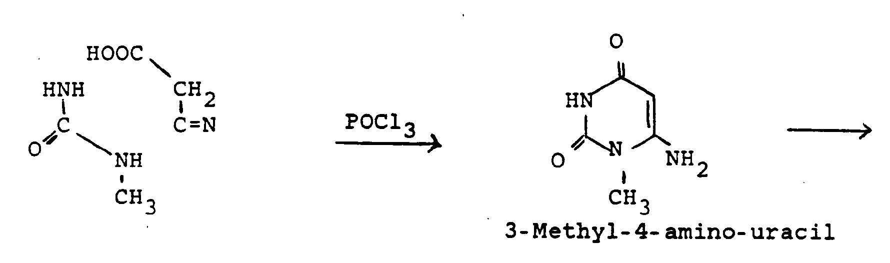

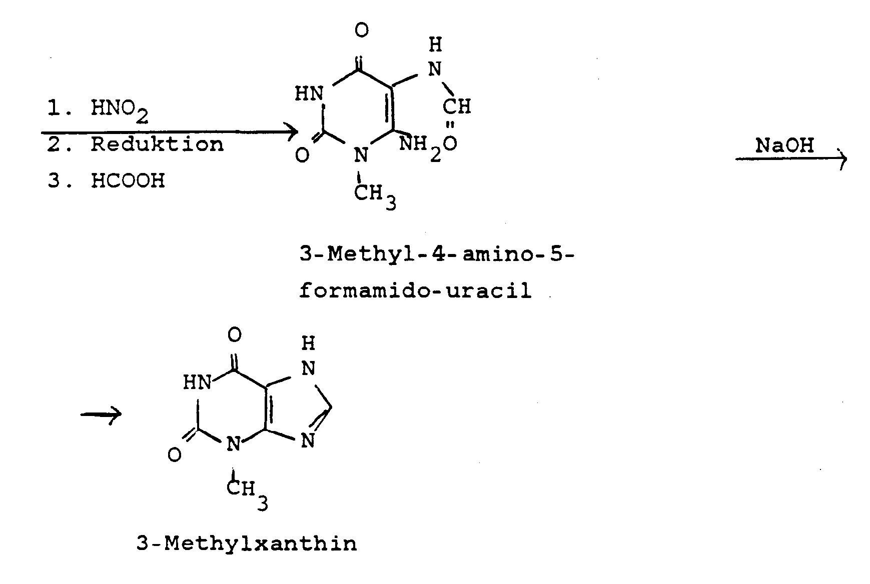

Bei dieser Synthese - vermutlich in deren letzter Stufe (der alkalischen Ringschlußreaktion) - bilden sich z.T. stark fluoreszierende Nebenprodukte, die den Xanthinen nach deren Ausfällung aus ihrer alkalischen Lösung hartnäckig anhaften und sich nur durch mehrfaches Umfällen oder Umkristallisieren mit entsprechenden Ausbeuteverlusten beseitigen lassen; die Ausfällung der Xanthine aus ihrer alkalischen Lösung wird üblicherweise durch anorganische oder organische Säuren wie z.B. von Schwefelsäure, Salzsäure oder Essigsäure durchgeführt.

Es wurde bereits versucht, durch Zugabe von Formaldehyd bei der alkalischen Ringschlußreaktion die Verunreinigung durch die fluoreszierenden Nebenprodukte zu vermindern (DD-A 21 220). Auch bei diesem Verfahren ist jedoch immer noch ein mindestens einmaliges Umkristallisieren erforderlich (vgl. Spalte 2, Zeilen 17/18 der DD-A).

In dem Bestreben, nach der vorerwähnten alkalischen Ringschlußreaktion gleich - d.h. ohne weitere Reinigungsoperationen - zu einem praktisch fluoreszenzfreien Xanthin zu gelangen bzw. ein bereits vorliegendes fluoreszierendes Xanthin mit möglichst geringem Aufwand und möglichst geringen Substanzverlusten von den fluoreszierenden Verunreinigungen zu befreien, wurde nun überraschend gefunden, daß dieses Ziel dadurch erreicht wird, daß man Xanthine, welche mindestens noch ein unsubstituiertes N-Atom im Molekül enthalten, aus wäßrig-alkalischer Lösung mittels CO₂ (Kohlendioxid) zur Ausfällung bringt.

Die Ausfällung mittels CO₂ kann direkt im Anschluß an die alkalische Ringschlußreaktion des entsprechenden Diaminouracilderivates zum Xanthin-Ringgerüst durchgeführt werden. Wenn ein bereits anderweitig erhaltenes fluoreszierendes Xanthin vorliegt, kann dieses auch in einer wäßrigalkalischen Lösung aufgelöst und daraus mittels CO₂ wieder ausgefällt werden. Die aus der wäßrig-alkalischen Lösung mittels CO₂ ausgefällten Xanthine sind frei bzw. praktisch frei von fluoreszierenden Nebenprodukten.

Der Effekt des erfindungsgemäßen Ausfällungsprozesses kommt möglicherweise dadurch zustande, daß die Ausfällung mittels CO₂ schonender und kontrollierter geschieht als mit den bisher zu diesem Zweck angewandten anorganischen und organischen Säuren. Dadurch könnten gleichmäßigere und geordnetere Kristalle gebildet werden, welche keine - oder jedenfalls praktisch keine - Verunreinigungen einschließen.

Außer den Vorteilen in Bezug auf Produktqualität und der Vermeidung von Ausbeuteverlusten, besitzt das erfindungsgemäße Ausfällungsverfahren auch noch einen ökologischen Vorteil, da die Mutterlaugen anstelle von Sulfaten, Chloriden oder Acetaten ökologisch unbedenkliches Carbonat enthalten und auch auf den Einsatz von Lösemitteln zur Entwässerung des filtrierten oder zentrifugierten Xanthinproduktes aufgrund der wesentlich verbesserten Kristallinität verzichtet werden kann.

Dem erfindungsgemäßen Verfahren sind im Prinzip alle Xanthine mit mindestens noch einem unsubstituierten N-Atom im Molekül zugänglich; d.s. - wegen des aziden Wasserstoffs am N - schwach saure Verbindungen, die sich in wäßrig-alkalischem Medium (NH₄OH, LiOH, NaOH, KOH, etc.) unter entsprechender Salzbildung lösen. Der pH-Wert des wäßrig-alkalischen Mediums liegt im allgemeinen bei mindestens etwa 10.

Bevorzugte Xanthine mit mindestens noch einem unsubstituierten N-Atom im Molekül sind diejenigen der folgenden Formel I 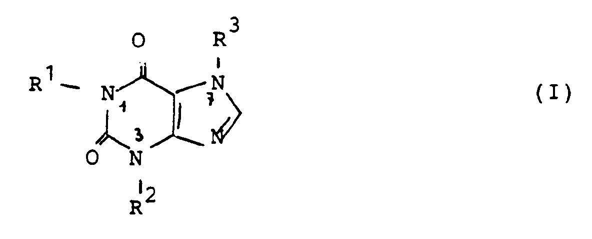
in welcher R¹, R² und R³ unabhängig voneinander = H oder (C₁-C₆)-Alkyl, vorzugsweise H oder CH₃,
wobei aber mindestens einer der 3 Reste = H sein muß.

Für das Verfahren besonders bevorzugte Xanthine sind 3-Methylxanthin (= Verbindung der Formel I mit R¹ = R³ = H und R² = CH₃).
1,3-Dimethylxanthin (= Verbindung der Formel I mit R¹ = R² = CH₃ und R³ = H; Theophyllin)
und
3,7-Dimethylxanthin (= Verbindung der Formel I mit R¹ = H und R² = R³ = CH₃; Theobromin).

In dem wäßrig-alkalischen Medium kann praktisch soviel Xanthin bis zur Sättigungsgrenze gelöst werden. Diese Lösung wird dann zwecks Ausfällung des Xanthins mit CO₂ in Kontakt gebracht. Dies kann vorteilhaft durch Einleiten von CO₂ in den Gasraum eines mit der wäßrig-alkalischen Xanthinlösung teilweise gefüllten Rührgefäßes unter intensivem Rühren der Lösung geschehen. Auch die Anwendung eines Begasungsrührers oder ein Umpumpen der Lösung bzw. Suspension, wobei nach Art einer Wasserstrahlpumpe ein intensiver Austausch zwischen der Flüssigkeit und dem mitgerissenen Gas erfolgt, ist günstig.

Zweckmäßig ist weiterhin auch die Zuführung des CO₂-Gases über eine Mengenregelung, um die Kristallwachstumsgeschwindigkeit optimal durch eine kontrollierte CO₂-Aufnahme zu steuern.

Der zur vollständigen Ausfällung erforderliche End-pH-Wert wird zweckmäßig über eine Druckregelung eingestellt und mit einer pH-Elektrode kontrolliert. Der genannte End-pH-Wert liegt durchweg noch im alkalischen Bereich (zwischen etwa 7 und 9,5) und variiert innerhalb dieses Bereiches geringfügig je nach dem jeweiligen Xanthin. Der zur vollständigen Ausfällung erforderliche End-pH-Wert liegt z.B. bei 3-Methylxanthin zwischen etwa 7,5 und 8,5 und bei 1,3-Dimethylxanthin (= Theophyllin) und 3,7-Dimethylxanthin (= Theobromin) zwischen etwa 8,8 und 9,2. Die jeweiligen pH-Werte sind durch einfache Routineversuche leicht feststellbar. Die Einstellung der End-pH-Werte kann vorteilhaft über den CO₂-Druck erfolgen. Zweckmäßig ist im allgemeinen ein CO₂-Überdruck von etwa 0,5 bis 6,0 bar.

Zur Erzielung einer optimalen Kristallbildung ist auch die Durchführung der Ausfällung bei erhöhter Temperatur von Vorteil, wobei ein Temperaturbereich von etwa 80 bis 110°C, insbesondere von etwa 90 bis 100°C, bevorzugt ist.

Nach beendeter Ausfällung wird das Produkt von der flüssigen Phase abgetrennt (z.B. über eine Kutsche oder Zentrifuge) und mit Wasser nachgewaschen. Das Produkt ist dann praktisch fluoreszenzfrei.

Das erfindungsgemäße Fällungsverfahren wird nun durch die folgenden Beispiele näher erläutert.

### Beispiel 1

500 g 3,7-Dimethylxanthin roh mit einem Gehalt von ca. 97 % wird in 3000 ml Wasser suspendiert und mit 114 g Natronlauge bei 100°C gelöst. Ca. 130 g Kohlendioxid werden bei einem Druck von 1,0 bis 1,5 bar innerhalb von 2 - 4 Stunden unter kräftigem Rühren in die Lösung eingeleitet. Nach Beendigung der Fällung wird der pH-Wert der Suspension über den CO₂-Druck auf einen Wert von 8,8 bis 9,2 eingestellt. Die Suspension wird abgekühlt auf 40°C und auf einer Nutsche das ausgefällte 3,7-Dimethylxanthin abgetrennt.

Ausbeute: 95 % d.Th., Gehalt: 99,9 %.

### Beispiel 2

650 kg 3-Methylxanthin roh als Natrium-Salz gelöst in 4200 kg Wasser werden auf 100°C erhitzt und innerhalb von 4 Stunden ca. 310 kg Kohlendioxid in die Kessel eingeleitet. Der Kesselinhalt wird während der Fällung im Kreis gepumpt und dabei im Gasraum des Rührkessels intensiv mit dem Kohlendioxid vermischt. Der CO₂-Überdruck im Kessel wird langsam innerhalb von 2 Stunden von 0 auf 4 bar erhöht, gesteuert über die CO₂-Menge. Nach Abkühlen auf 45°C wird das 3-Methylxanthin auf einer Zentrifuge abgetrennt.

Ausbeute: 610 kg 3-Methylxanthin entsprechend 94 % d.Th.. Die Fluoreszenz liegt um den Faktor 100 niedriger als bei 3-Methylxanthin, das unter vergleichbaren Bedingungen mit Schwefelsäure gefällt wurde.

## Patentansprüche

1. Verfahren zur Gewinnung annähernd fluoreszenzfreier Xanthine,
dadurch gekennzeichnet, daß man Xanthine, welche mindestens noch ein unsubstituiertes N-Atom im Molekül enthalten, aus wäßrig-alkalischer Lösung mittels CO₂ zur Ausfällung bringt.

2. Verfahren nach Anspruch 1, dadurch gekennzeichnet, daß man als Xanthine, welche noch mindestens ein unsubstituiertes N-Atom im Molekül enthalten, Xanthine der folgenden Formel I einsetzt: 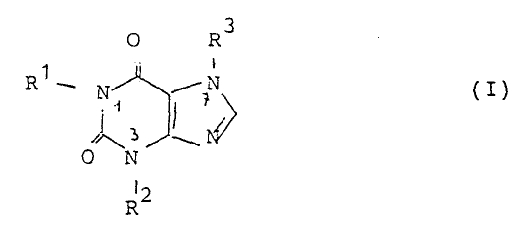 in welcher R¹, R² und R³ unabhängig voneinander H oder (C₁-C₆)-Alkyl bedeuten, wobei aber mindestens einer der 3 Reste = H sein muß.

3. Verfahren nach Anspruch 2, dadurch gekennzeichnet, daß R¹, R² und R³ unabhängig voneinander H oder CH₃ bedeuten, wobei aber mindestens einer der Reste H sein muß.

4. Verfahren nach Anspruch 1 oder 2, dadurch gekennzeichnet, daß man
3-Methylxanthin,
1,3-Dimethylxanthin oder
3,7-Dimethylxanthin zur Ausfällung bringt.

5. Verfahren nach einem oder mehreren der Ansprüche 1 bis 4, dadurch gekennzeichnet, daß man für die Ausfällung einen End-pH-Wert zwischen etwa 7 und 9,5 einstellt.

6. Verfahren nach einem oder mehreren der Ansprüche 1 bis 5, dadurch gekennzeichnet, daß man die Ausfällung bei CO₂-Überdrucken zwischen 0,5 und 6 bar durchführt.

7. Verfahren nach einem oder mehreren der Ansprüche 1 bis 6, dadurch gekennzeichnet, daß man die Ausfällung bei Temperaturen zwischen 80 und 110°C durchführt.

8. Verfahren nach Anspruch 7, dadurch gekennzeichnet, daß man die Ausfällung bei Temperaturen zwischen 90 und 100°C durchführt.

## Claims

1. A process for obtaining almost fluorescence-free xanthines, which comprises making xanthines which still contain at least one unsubstituted nitrogen atom in the molecule precipitate from aqueous-alkaline solution by means of CO₂.

2. The process as claimed in claim 1, wherein xanthines of the following formula I: 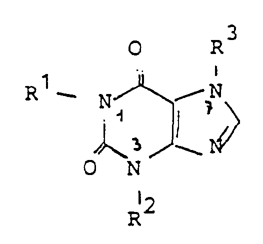 in which R¹, R² and R³ independently of one another are H or (C₁-C₆)-alkyl,
where, however, at least one of the 3 radicals must be H, are employed as xanthines which still contain at least one unsubstituted nitrogen atom in the molecule.

3. The process as claimed in claim 2, wherein R¹, R² and R³ independently of one another are H or CH₃, where, however, at least one of the radicals must be H.

4. The process as claimed in claim 1 or 2, wherein 3-methylxanthine,
1,3-dimethylxanthine or
3,7-dimethylxanthine is made to precipitate.

5. The process as claimed in one or more of claims 1 to 4, wherein a final pH of between about 7 and 9.5 is set for the precipitation.

6. The process as claimed in one or more of claims 1 to 5, wherein the precipitation is carried out at CO₂ excess pressures between 0.5 and 6 bar.

7. The process as claimed in one or more of claims 1 to 6, wherein the precipitation is carried out at temperatures between about 80 and 110°C.

8. The process as claimed in claim 7, wherein the precipitation is carried out at temperatures between 90 and 100°C.

## Revendications

1. Procédé pour l'obtention de xanthines pratiquement exemptes de fluorescence, caractérisé en ce que l'on fait précipiter à l'aide de CO₂, dans une solution aqueuse-alcaline, des xanthines qui comportent encore au moins un atome d'azote non substitué dans leur molécule.

2. Procédé selon la revendication 1, caractérisé en ce que, en tant que xanthines qui comportent encore au moins un atome d'azote non substitué dans leur molécule, on utilise des xanthines de formule I suivante: 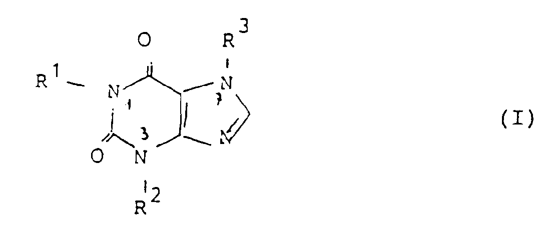 dans laquelle R¹, R² et R³ représentent, indépendamment les uns des autres, un atome d'hydrogène ou un groupe alkyle en C₁-C₆,
au moins un des trois radicaux devant toutefois être un atome d'hydrogène.

3. Procédé selon la revendication 2, caractérisé en ce que R¹, R² et R³ représentent, indépendamment les uns des autres, H ou CH₃, au moins un des radicaux devant toutefois être un atome d'hydrogène.

4. Procédé selon la revendication 1 ou 2, caractérisé en ce que l'on fait précipiter
la 3-méthylxanthine,
la 1,3-diméthylxanthine ou
la 3,7-diméthylxanthine.

5. Procédé selon une ou plusieurs des revendications 1 à 4, caractérisé en ce que, pour la précipitation, on ajuste un pH final compris entre environ 7 et 9,5.

6. Procédé selon une ou plusieurs des revendications 1 à 5, caractérisé en ce que l'on effectue la précipitation sous des surpressions de CO₂ comprises entre 0,5 et 6 bars.

7. Procédé selon une ou plusieurs des revendications 1 à 6, caractérisé en ce que l'on effectue la précipitation à des températures comprises entre 80 et 110°C.

8. Procédé selon la revendication 7, caractérisé en ce que l'on effectue la précipitation à des températures comprises entre 90 et 100°C.
